# EUROPÄISCHE PATENTSCHRIFT

(11) **EP 2 062 608 B1**
(45) Veröffentlichungstag und Bekanntmachung des Hinweises auf die Patenterteilung: **01.05.2013**
(21) Anmeldenummer: 08169011.7
(22) Anmeldetag: 13.11.2008
(51) Int. Cl.: A61M 15/00, A61J 1/00, A61J 1/06, B65D 1/09

(54) **Einwegampulle für eine Vorrichtung zur Erzeugung von Aerosolen**
Single use ampoule for a device to create aerosols
Ampoule unidirectionnelle pour un dispositif de production d'aérosols

(30) Priorität: 23.11.2007 DE 102007056462
(43) Veröffentlichungstag der Anmeldung: 27.05.2009
(73) Patentinhaber: PARI Pharma GmbH, 82319 Starnberg (DE)
(72) Erfinder: Lönner, Mihaela, 85586, Poing (DE); Gallem, Thomas, 81371, München (DE); Hetzer, Uwe, 81369, München (DE)
(74) Vertreter: HOFFMANN EITLE

(56) Entgegenhaltungen:
- EP-A- 0 326 391
- DE-A1- 3 823 428
- DE-A1- 4 306 458
- DE-A1-102005 038 619
- US-A- 3 171 412
- US-A- 4 671 763
- US-B1- 6 197 260

## Beschreibung

Die vorliegende Erfindung betrifft eine Einwegampulle für den Einsatz in einer Vorrichtung, die zur Erzeugung von Aerosolen zur topischen Applikation auf die Haut oder in Körperhöhlen, wie z.B. Nase und Lunge verwendet werden kann, um Krankheiten von Mensch und Tier zu diagnostizieren, vorzubeugen oder zu behandeln. Im wesentlichen handelt es sich um eine Einwegampulle, die mit einem flüssigen Medikament befüllt ist und erst in der Vorrichtung geöffnet wird, um das flüssige Medikament einem Aerosolerzeuger zuzuführen, der so ausgestaltet sein kann, dass ein Aerosol kontinuierlich, getaktet oder atemzuggesteuert erzeugt und abgegeben wird, um damit Krankheiten diagnostizieren, vorzubeugen oder behandeln zu können.

Eine derartige Einwegkartusche ist aus der DE 10 2005 083 619 A1 bekannt. Die dort beschriebene Einwegampulle umfasst einen Medikamentenbehälter, der ein Medikament enthält und aus einem Behälterkörper und einem Behälterboden gebildet ist sowie eine den Behälterboden umgebende Sollbruchstelle. Mittels eines in der Vorrichtung zur Aerosolerzeugung global vorgesehenen Dorns wird der Behälterboden entlang der Sollbruchstelle aufgestochen, um die Einwegampulle zu öffnen und das flüssige Medikament dem Aerosolerzeuger zuzuführen.

Die Aufgabe der vorliegenden Erfindung bestand nun darin diese bekannte Einwegampulle derart weiterzubilden, dass sie sich leicht und ohne Gefahr einer Beschädigung handhaben sowie in die Vorrichtung zur Aerosolerzeugung einsetzen lässt, dass beim Öffnungsvorgang der Ampulle während des Einsetzens kein Medikament entweichen kann und auch während der Aerosolerzeugung die Schnittstelle zwischen der Vorrichtung zu Aerosolerzeugung und Ampulle abgedichtet ist, so dass eine Kontamination oder ein unbeabsichtigtes Entweichen von Medikament verhindert wird.

Diese Aufgabe wird gemäß der vorliegenden Erfindung durch eine Einwegampulle mit den Merkmalen des Patentanspruchs 1 gelöst. Vorteilhafte Weiterbildungen der vorliegenden Erfindung sind in den abhängigen Patentansprüchen genannt.

Der vorliegenden Erfindung liegt der Gedanke zu Grunde den Behälterboden sowie die den Behälterboden wenigstens teilweise umgebende Sollbruchstelle durch einen schützenden Kragen zu umschließen, so dass ein versehentliches Öffnen bzw. Aufbrechen der Ampulle außerhalb der Vorrichtung zu Aerosolerzeugung durch eine Beschädigung der Sollbruchstelle verhindert werden kann. Neben dieser Funktion hat sich auf vorteilhafter Weise ebenso ergeben, dass der Kragen zur Abdichtung während des und ggf. auch nach dem Öffnungsvorgang(-s) sowie zur Führung der Ampulle bei diesem Vorgang genutzt werden kann.

Dementsprechend umfasst die Einwegampulle zum Einsetzen in eine Vorrichtung zu Aerosolerzeugung gemäß der vorliegenden Erfindung einen Medikamentenbehälter, der ein Medikament, insbesondere ein flüssiges Medikament enthält und aus einem Behälterkörper und einem Behälterboden gebildet ist sowie eine den Behälterboden zumindest teilweise umgebende Sollbruchstelle. Die erfinderische Einwegampulle kennzeichnet sich durch einen Kragen, der die Sollbruchstelle nach außen hin umgibt und den Behälterkörper über den Behälterboden hinaus verlängert. Damit liegt der Behälterboden und die ihn umgebende Sollbruchstelle in einem Abstand von der Stirnseite der Einwegampulle nach hinten versetzt und ist damit durch den Kragen geschützt. Dieser Versatz bewirkt neben dem Schutz ferner den Vorteil, dass eine Innenfläche des Kragens während des Öffnungsvorgangs der Einwegampulle in die Vorrichtung zu Aerosolerzeugung, z. B. auf einen Dorn, mit einem Dichtelement in Eingriff bringbar ist, z. B. einem O-Ring oder einer Dichtungskante, der bzw. die den Dorn umgibt, während die Einwegampulle noch nicht geöffnet ist, so dass auch während des Öffnungsvorgangs eine Abdichtung gewährleistet ist. Darüber hinaus übernimmt der Kragen durch seine Ausgestaltung eine Führungsfunktion und unterstützt damit eine saubere Positionierung der Ampulle während des Öffnungsvorgangs.

Vorteilhafterweise weitet sich die innen liegende Fläche des Kragens von dem Behälterboden ausgehend konisch auf. Hierfür kann der gesamte Kragen in Form eines hohlen Kegelstumpfes ausgestaltet sein. Diese Ausgestaltung unterstützt auf vorteilhafter Weise die Führungsfunktion des Kragens und das in Eingriff bringen mit einem möglichen zusätzlichen Dichtungselement am Aufstechelement (Dorn) der Vorrichtung zu Aerosolerzeugung.

Des Weiteren ist es bevorzugt den Behälterkörper in zwei Funktionsbereiche zu unterteilen, so dass die Ampulle modular aufgebaut ist. Hier hat es sich als besonders vorteilhaft herausgestellt, wenn ein erster Funktionsbereich, der den Behälterboden umfasst, hinsichtlich des Leerlaufverhaltens der Ampulle geometrisch gestaltet ist und bei zumindest allen Ampullen, die für die gleiche Vorrichtung zu Aerosolerzeugung bestimmt sind, unabhängig von der Größe und Füllmenge gleich bleibt, wohingegen der zweite Funktionsbereich im Wesentlichen zylindrisch ausgebildet ist und in Anpassung an verschiedene Füllmengen entsprechend unterschiedlich lang ausgestaltet wird. Dies ermöglicht insbesondere die Verwendung der gleichen Vorrichtung zu Aerosolerzeugung mit im Wesentlichen gleichen Ampullen, die sich ausschließlich in ihre Länge voneinander unterscheiden, was sowohl aus herstellungstechnischen Gründen wie auch aus dem Blickwinkel der Benutzerfreundlichkeit vorteilhaft ist. Der erste Funktionsbereich der Ampulle, der der Vorrichtung zu Aerosolerzeugung zugewandt ist und damit den Behälterboden umfasst, wird dabei so gestaltet sein, dass das Medikament mit einer reproduzierbaren Dosisgenauigkeit dem Aerosolerzeuger zuführbar ist, wobei unter anderem die verschiedenen Haltewinkel der Vorrichtung zu Aerosolerzeugung bei der Therapie zu berücksichtigen sind. Dabei hat es sich als vorteilhaft herausgestellt, wenn dieser erste Funktionsbereich zumindest im Inneren der Ampulle zum Behälterboden hin konisch, d. h. trichterförmig zuläuft, wobei der Winkel zwischen Behälterboden und der trichterförmigen Seitenwand vorzugsweise in einem Bereich zwischen ca. 105° und 125° und bevorzugterweise bei ca. 110° liegt. Darüber hinaus kann die Ampulle, insbesondere aber dieser erste Funktionsbereich auch bei Ampullen, die für unterschiedliche Vorrichtung zu Aerosolerzeugungen gedacht sind, unterschiedlich ausgestaltet sein und zwar derart, dass jeweils nur die Ampulle, die für die jeweilige Vorrichtung zu Aerosolerzeugung bestimmt ist, in eine solche eingesetzt werden kann und/oder die Vorrichtung zu Aerosolerzeugung nur mit vorbestimmter Ampulle funktionsfähig ist. Diese Art der Identifizierung bzw. Verschlüsselung kann z. B. durch eine oder mehrere Verschlüsselungselemente realisiert sein. Sie können z. B. aus einem oder mehreren Vorsprüngen bestehen, die sich von dem Ampullenkörper erstrecken. Diese Vorsprünge, die in zugehörige Nuten in der dafür vorgesehenen Vorrichtung zu Aerosolerzeugung passen, ermöglichen das Einführen der Ampulle oder verhindern es, wenn nicht die richtige Kombination vorliegt. Auch können die einen oder mehreren Vorsprünge dazu genutzt werden einen elektrischen Schalter zu betätigen, um einen Kreis in der Vorrichtung zu Aerosolerzeugung zu schließen. Erst bei geschlossenem Kreis könnte beispielsweise die Stromversorgung des Aerosolgenerators bzw. Aerosolerzeugers erfolgen. Alternativ können die Verschlüsselungselemente derart ausgestaltet sein, dass sie den Betrieb der Vorrichtung zu Aerosolerzeugung verhindern, es sei denn sie sind als durch die Vorrichtung zu Aerosolerzeugung richtig erkannt. Z. B. kann der Behälterkörper ein lesbares Muster, wie beispielsweise einen Barcode, ein magnetisches Muster oder ähnliches umfassen, das durch die Vorrichtung zu Aerosolerzeugung ausgelesen und bestätigt werden muss bevor der Betrieb zugelassen wird.

Um die Ampulle sicher in die Vorrichtung zu Aerosolerzeugung einsetzen zu können und die leere Ampulle einfach wieder herausnehmen zu können, ist eine Befestigungsnut, die vorzugsweise ringförmig um die Ampulle verläuft in Längsrichtung der Ampulle mittig oder näher zum Behälterboden vorgesehen. Die Position in der Mitte der Ampulle bzw. näher zum Behälterboden hat dabei den Vorteil, dass die zum Öffnen der Ampulle erforderlichen Kräfte, die beim Durchbrechen der Sollbruchstelle mittels des Aufstechelements (Dorn) auftreten, möglichst nahe im Bereich der Krafteinleitung aufgenommen werden können. Ist die Ampulle, wie zuvor beschrieben, in zwei Funktionsbereiche unterteilt, so hat es sich als vorteilhaft erwiesen, wenn die Befestigungsnut die Ampulle in die zwei Funktionsbereiche unterteilt.

Um die Ampulle zusätzlich zu versteifen und einer Deformation während des Öffnungsvorgangs entgegenzuwirken, ist wenigstens eine Verstärkungsrippe auf dem Behälterkörper vorgesehen. Bevorzugterweise sind zwei diametral gegenüberliegende Verstärkungsrippen vorgesehen. Diese erstrecken sich vorzugsweise von der Stirnseite der Ampulle, d. h. über wenigstens einen Teilbereich des Kragens, über den Behälterboden hinaus und über einen Teilbereich des Behälterkörpers. Dabei ist es besonders bevorzugt, wenn die Verstärkungsrippen bei Unterteilung in zwei Funktionsbereiche nur im ersten Funktionsbereich bzw. bei vorhandener Befestigungsnut auf der Seite der Befestigungsnut, die den Behälterboden umfasst, vorgesehen sind.

Darüber hinaus ist es erforderlich die Ampulle derart sichtbar zu kennzeichnen, dass Medikament, Chargennummer und Verfallsdatum erkennbar sind. Dies wird erfindungsgemäß dadurch gelöst, dass gegenüber dem Behälterboden eine Fahne an dem Behälterkörper vorgesehen ist, die sich vorzugsweise in entgegengesetzter Richtung wie der Kragen von dem Behälterkörper weg erstreckt. Diese Fahne weist zwei entgegengesetzte flächenhafte Seiten auf, auf denen die notwendigen Informationen aufgebracht werden können. Diese Fahne kann zusätzlich dazu genutzt werden die Entnahme der Ampulle zu erleichtern, d. h. eine Grifflasche bilden, die der Anwender greifen kann, um die Ampulle aus der Vorrichtung zu Aerosolerzeugung zu drücken oder zu ziehen. Dies ist insbesondere bei kurzen Ampullen für kleine Füllmengen (siehe modularer Aufbau) nützlich. Zu diesem Zweck ist die Vorrichtung zu Aerosolerzeugung vorzugsweise derart ausgestaltet, dass die Fahne zum Entfernen der Ampulle frei liegt. Dabei ist es insbesondere auch bevorzugt, wenn die Fahne bei in die Vorrichtung zu Aerosolerzeugung eingesetzter Ampulle aus der Vorrichtung zu Aerosolerzeugung vorragt, so dass die zuvor erwähnten Informationen auch in diesem Fall sichtbar bleiben.

Gemäß einer Ausführungsform der vorliegenden Erfindung ist die Ampulle einstückig ausgebildet, wobei es sich bevorzugterweise um eine Blow-Fill-Seal-Ampulle handelt, was bedeutet, dass die Ampulle im sog. Blow-Fill-Seal-Verfahren (Blasen-Füllen-Verschließen) hergestellt ist. Diese Verfahrenstechnik ist z. B. durch die DE 38 33 036, DE 38 23 428 und die US 4,671,763, 9,319,374 und 4,995,511 bekannt geworden, so dass der Fachmann bezüglich der Verfahrenstechnik als solches auf diese Schriften verwiesen wird. Vorzugweise besteht die Ampulle aus Polyethylen, Polypropylen oder einem thermoplastischen Copolymer.

Schließlich kann die einstückige oder aber auch mehrstückige Ampulle mit einem zusätzlichen Element versehen sein oder versehen werden, wobei es sich bei diesem zusätzlichen Element um ein separates Dichtelement handelt, das in den Kragen eingesetzt wird, um beim Einsetzen der Ampulle in die Vorrichtung zu Aerosolerzeugung und insbesondere beim Öffnungsvorgang eine Abdichtung zwischen Krageninnenfläche und Aufstechelement (z. B. Dorn) zu bewirken.

Darüber hinaus ist es zur Vermeidung der Bildung von Luftblasen beim Ausströmen des Medikaments bevorzugt, dass der Öffnungsdurchmesser der Ampulle, der sich aus dem Durchmesser des Behälterbodens und dem Durchmesser und zumindest einem Teil der Sollbruchstelle zusammensetzt größer als ca. 8 mm ist und vorzugsweise in einem Bereich zwischen ca. 8 mm und ca. 15 mm liegt. Gemäß einer besonders bevorzugten Ausführungsform beträgt der Öffnungsdurchmesser ca. 10 mm. Entsprechend ist es bevorzugt, dass das Aufstechelement der Vorrichtung zu Aerosolerzeugung, z. B. der Dorn, einen Innendurchmesser von mindestens ca. 8 mm haben muss.

Der Medikamentenbehälter der oben beschriebenen Ampulle enthält vorzugsweise bis zu 10 ml, am meisten bevorzugt zwischen 0,25-5 ml Medikament. In diesem Voluminabereich ist es durch Dimensionierung und Ausgestaltung insbesondere des zweiten Funktionsbereichs, wie es oben erläutert wurde, möglich Volumina mit einer Genauigkeit von ± 25% bis ± 5% des Sollwertvolumens genau zu dosieren.

Gemäß einer bevorzugten Ausführungsform enthält das in der Ampulle enthaltene Medikament wenigstens einen Wirkstoff und vorzugsweise wenigstens einen Hilfsstoff in gelöster oder suspendierter Form. Bei dem Medikament handelt es sich vorzugsweise um ein Medikament zur Diagnose, Prophylaxe oder Behandlung von Krankheiten bei Mensch und Tier in vernebelter Form, wobei das Medikament in Verbindung mit einer perforierten schwingenden Membran zu Tröpfchen mit einem mittleren Durchmesser < 6µm vernebelbar ist bzw. als pulsierendes Aerosol mit einem solchen mittleren Massendurchmesser vernebelt werden kann. Bezüglich der möglichen verwendbaren Medikamente und Zusatz- und Hilfsstoffe wird auf die nachfolgende Beschreibung verwiesen. Dementsprechend betrifft die vorliegende Erfindung ferner die Verwendung einer erfindungsgemäßen Ampulle in einem Aerosolerzeuger, wobei deren Medikament von einem Aerosolerzeuger vernebelt wird und zur lokalen, nasalen oder pulmonalen Applikation verwendet wird.

Weitere Vorteile und Merkmale der vorliegenden Erfindung werden aus der folgenden Beschreibung einer bevorzugten Ausführungsform ersichtlich, die unter Bezugnahme auf die begleitenden Zeichnungen erfolgt.

In den Zeichnungen zeigen:
Fig. 1 eine erfindungsgemäße Einwegampulle in einer perspektivischen Darstellung;
Fig. 2a und 2b jeweils einen Querschnitt durch die erfindungsgemäße Ampulle, wobei Fig. 2a einen Querschnitt parallel zu den und durch die Verstärkungsrippen sowie der bzw. die Fahne darstellt und Fig. 2b einen Querschnitt senkrecht zu dem Querschnitt aus Fig. 2a zeigt;
Fig. 3 eine perspektivische Darstellung eines Teils einer Vorrichtung zu Aerosolerzeugung mit eingesetzter Ampulle;
Fig. 4 einen Querschnitt durch die in Fig. 3 dargestellte Anordnung;
Fig. 5 eine Vergrößerung der Schnittstelle zwischen Ampulle und Dorn aus Fig. 4; und
Fig. 6 die in Fig. 5 dargestellte Schnittstelle mit geöffnetem Behälterboden, d. h. bei eingesetzter Ampulle.

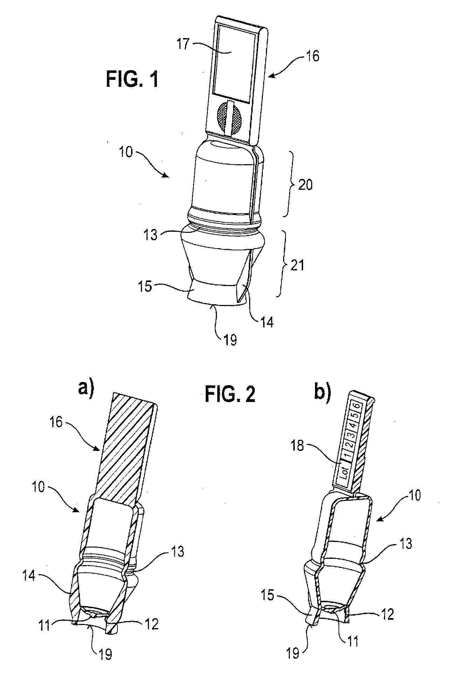

Fig. 1 zeigt eine erfindungsgemäße Einwegampulle umfassend einen Medikamentenbehälter, der ein flüssiges Medikament (nicht dargestellt) enthält und aus einem Behälterkörper 10 und einem Behälterboden 11 (siehe Fig. 2) aufgebaut ist. Der Behälterkörper 10 ist im Wesentlichen hohlzylindrisch aufgebaut und der Behälterboden 11 weist eine im Wesentlichen kreisförmige Gestalt mit einem Durchmesser (inkl. einer Sollbruchstelle 12) größer als ca. 8 mm auf, um die Bildung von Luftblasen zu verhindern, die ein Nachströmen des Medikaments behindern könnten. Vorzugsweise liegt der Durchmesser bei ca. 10 mm. Zwischen dem Behälterboden 11 und dem Behälterkörper 10 ist die Sollbruchstelle 12 ausgebildet. Die Sollbruchstelle 12 umgibt den Behälterboden 11 zumindest teilweise, vorzugsweise und wie dargestellt jedoch vollständig und ist ringförmig in ihrer Form. Die Sollbruchstelle 12 wird beispielsweise durch eine Materialschwächung bewirkt, d. h. die Materialstärke der Sollbruchstelle 12 ist im Vergleich zu der Materialstärke des Behälterbodens 11 reduziert.

Ferner weist die erfindungsgemäße Ampulle, wie es in den Fig. 1 und 2 dargestellt ist, einen Kragen 15 auf, der im Querschnitt ebenfalls ringförmig ausgestaltet ist und den Behälterkörper 10 über den Behälterboden 12 hinaus verlängert. Dadurch sitzt der Behälterboden in einem Abstand von der Stirnseite 19 und auch die ihn umgebende Sollbruchstelle 12 ist durch den Kragen 15 vor Beschädigungen und einem möglichen Öffnen vor dem Gebrauch geschützt.

Der Kragen 15 ist dabei vom Behälterboden ausgehend derart ausgestaltet, dass er bezüglich seiner Innenkontur im Querschnitt größer wird, d. h. er ist konisch ausgebildet, wobei er sich vom Behälterboden 11 ausgehend aufweitet (der Durchmesser des ringförmigen Querschnitts wird vom Behälterboden 11 ausgehend größer.

Wie es in Fig. 1 angedeutet ist, weist der Behälterkörper 10 in Längsrichtung etwas näher zum Behälterboden 11 eine ringförmig umlaufende Befestigungsnut 13 auf, die der Befestigung (Halterung bzw. Fixierung) der Ampulle in einer Vorrichtung zu Aerosolerzeugung (siehe später) dient. Diese Befestigungsnut unterteilt den Behälterkörper in einen ersten und einen zweiten Funktionsbereich. Der erste Funktionsbereich 21 umfasst neben dem Kragen 15 einen Abschnitt des Behälterkörpers 10, der in seiner geometrischen Form derart gestaltet ist, dass das in der Ampulle enthaltene Medikament (nicht dargestellt) der Vorrichtung zu Aerosolerzeugung und insbesondere dessen Aerosolerzeuger mit einer reproduzierbaren Dosisgenauigkeit zugeführt werden kann. Zu diesem Zweck läuft der erste Funktionsbereich 21 zum Behälterboden konisch zu, d. h. seine Innenkontur ist zum Behälterboden 11 hin trichterförmig ausgebildet. Der zweite Funktionsbereich 20 ist im Wesentlichen hohlzylinderförmig ausgestaltet. Diese Ausgestaltung ermöglicht einen modularen Aufbau der erfindungsgemäßen Ampulle. So ist der Funktionsbereich 21 vorzugsweise bei allen Ampullen unabhängig von der Füllmenge gleich, so dass die Schnittstelle zwischen Ampulle und Vorrichtung zu Aerosolerzeugung unverändert bleiben kann, d. h. unterschiedliche Ampullen in die gleiche Vorrichtung zu Aerosolerzeugung einsetzbar sind. Darüber hinaus können die Ampullen auch derart ausgestaltet sein, dass sie nur für bestimmte Vorrichtung zu Aerosolerzeugungen geeignet sind. So können beispielsweise Ampulle für eine Vorrichtung zu Aerosolerzeugung so ausgestaltet sein, dass sie nicht in eine andere Vorrichtung zu Aerosolerzeugung passen und umgekehrt. Hierfür können sog. Verschlüsselungselemente bzw. Identifikationselemente vorgesehen sein, die z. B. Vorsprünge oder aber Barcodes oder magnetische Informationen beinhalten können. Die Vorsprünge können z. B. das Einsetzen der Ampulle in die Vorrichtung zu Aerosolerzeugung verhindern, wenn sie nicht für die entsprechende Vorrichtung zu Aerosolerzeugung bestimmt ist. Andererseits können die Vorsprünge auch mit einem elektrischen Schalter in Kontakt kommen, wenn die Ampulle in die richtige Vorrichtung zu Aerosolerzeugung eingesetzt ist, um den Betrieb der Vorrichtung zu Aerosolerzeugung zu gewährleisten. Gleichermaßen funktioniert auch das Auslesen der Informationen des Barcodes oder der magnetischen Informationen, die bei korrekter Ampulle einen Betrieb der Vorrichtung zu Aerosolerzeugung ermöglichen. Diese Elemente sind vorzugsweise gleichermaßen im ersten Funktionsbereich angeordnet. Der zweite Funktionsbereich 20 hingegen kann in seiner Größe in Längsrichtung variieren. Mit anderen Worten kann in Anpassung an die Füllmenge der jeweiligen Ampulle der Funktionsbereich 20 länger, d. h. mit einem größeren Volumen oder aber kürzer, d. h. mit einem geringeren Volumen, ausgestaltet werden. Dies ist sowohl aus fertigungstechnischen Gründen zum Herstellen unterschiedlicher Ampullen sinnvoll, andererseits jedoch auch hinsichtlich der Bedienerfreundlichkeit, weil der Benutzer die Ampullen unabhängig von ihrer Größe stets auf die gleiche Art und Weise in die Vorrichtung zu Aerosolerzeugung einsetzen wird. Auch deren Bedienung bleibt gleich. Schließlich ermöglicht diese Ausgestaltung die Verwendung verschiedenartige Ampullen, d. h. mit verschiedenartigen Füllmengen in ein und derselben Vorrichtung zu Aerosolerzeugung.

Darüber hinaus weist die erfindungsgemäße Ampulle im ersten Funktionsbereich 21 zwei diametral gegenüberliegende Verstärkungsrippen auf. Diese dienen der Dimensionsstabilität der Ampulle insbesondere beim Öffnungsvorgang, bei dem die Ampulle auf einen Dorn (siehe später) gedrückt wird, um den Behälterboden 11 entlang der Sollbruchstelle 12 aufzustechen. In der in Fig. 1 dargestellten Ausführungsform erstrecken sich die Verstärkungsrippen 14 über einen Teilbereich des Kragens 15 sowie einen Teilbereich des trichterförmig zulaufenden Abschnitts des Behälterkörpers 10.

Ferner ist an dem den Behälterboden 11 entgegengesetzten Ende des Behälterkörpers 10 eine Fahne 16 vorgesehen. Diese Fahne, die eine im Wesentlichen ebene Ausgestaltung mit zwei entgegengesetzten Flächenbereichen ausweist, ermöglicht die Anbringung eines Beschriftungsfeldes 17, das Informationen über das aufgenommene Medikament enthalten kann. Auf der entgegengesetzten Fläche können die Chargennummer 18 und das Haltbarkeitsdatum vorgesehen sein.

Wie es aus den Fig. 1 und 2 ersichtlich ist, ist die erfindungsgemäße Ampulle einstückig hergestellt. Dies erfolgt vorteilhafterweise im Blow-Fill-Seal-Verfahren (Blasen-Füllen-Verschließen). Bezüglich dieses Verfahrens wird der Fachmann auf die zuvor erwähnten Veröffentlichungen des Standes der Technik verwiesen. Die Ampulle kann aus Polyethylen, Polypropylen oder einem Copolymer hergestellt sein. Der Inhalt wird in einem variablen, aber vorher definierbaren Volumenbereich von ca. 0.25 ml bis 5 ml liegen und einem Aerosolerzeuger zugeführt werden, so dass ein Medikament vernebelt und zur topischen Applikation auf die Haut oder in Körperhöhlen, wie z.B. Nase und Lunge verwendet werden kann, um Krankheiten von Mensch und Tier zu diagnostizieren, vorzubeugen oder zu behandeln.

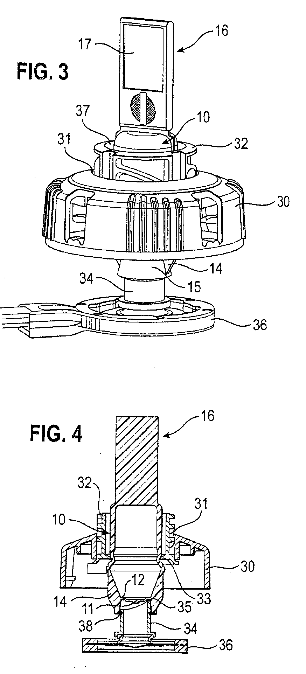

In Fig. 3 ist ein Teil einer Vorrichtung zu Aerosolerzeugung dargestellt. Diese umfasst wenigstens einen Deckel 30, der von dem Körper der Vorrichtung zu Aerosolerzeugung abnehmbar, z. B. abschraubbar, gestaltet ist. In diesem Deckel ist eine Durchgangsöffnung 31 vorgesehen, in die ein erstes Element 32 des Öffnungsmechanismus eingesetzt ist. Am Innenumfang der Öffnung 31 des Deckels 30 sind beispielsweise Nasen vorgesehen, die in Gewindenuten in dem Element 32 eingreifen oder umgekehrt. Durch eine Drehbewegung des Deckels 30 beim Anbringen des Deckels 30 auf dem Körper (nicht dargestellt) der Vorrichtung zu Aerosolerzeugung bewirkt der Eingriff der Nasen des Deckels 30 mit den Gewindenuten des Elements 32 eine translatorische Bewegung des Elements 32. Bezüglich dieser Ausgestaltung wird insbesondere auf die DE 10 2005 038 619 A1 verwiesen.

Des Weiteren umfasst das Element 32 Rastnasen 33, mit denen die Befestigungsnut 13 der erfindungsgemäßen Ampulle in Eingriff gebracht werden kann, um die Ampulle in dem Element 32 festzulegen.

Darüber hinaus umfasst die Vorrichtung zu Aerosolerzeugung einen Dorn 34, der hohlzylindrisch ausgebildet ist und an seinem einen Ende eine Schneidkante 35 aufweist. Der Innendurchmesser des Dorns 34 ist größer als 8 mm, um der Bildung von Luftblasen beim Ausströmen des Medikaments entgegenzuwirken, die das Nachlaufen des Medikaments behindern könnten. An dem entgegengesetzten Ende des Dorns 34 sitzt der Aerosolgenerator, bei dem es sich vorzugsweise um eine piezoelektrisch aktuierte Membran handelt. In dem Element 32 ist konzentrisch zu der Öffnung 31 des Deckels 30 vorzugsweise ebenfalls eine Durchgangsöffnung 37 ausgebildet, durch die die Ampulle im eingesetzten Zustand aus der Vorrichtung zu Aerosolerzeugung vorragt.

Ferner ist im oberen Bereich des Dorns 34 ein weiteres Dichtelement in Form eines O-Rings 38 vorgesehen.

Im Folgenden wird unter Bezugnahme auf die Fig. 3-6 die Verwendung einer erfindungsgemäßen Ampulle erläutert.

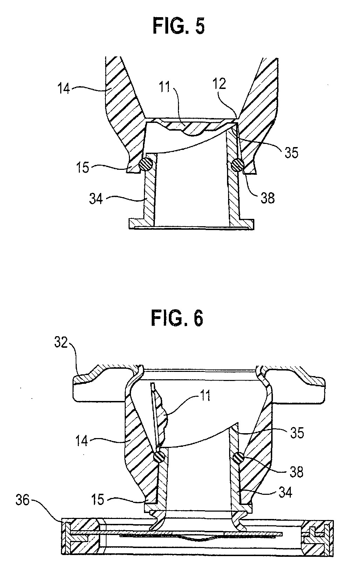

Soll eine Ampulle in die Vorrichtung zu Aerosolerzeugung eingesetzt werden, wird der Deckel 30 vom Körper der Vorrichtung zu Aerosolerzeugung abgenommen, z. B. abgeschraubt. Dabei bewegt sich das Element 32 translatorisch nach oben durch die Öffnung 31 des Deckels 30. Ist der Deckel abgenommen, so wird die Ampulle von der der Öffnung 37 des Elements 32 entgegengesetzten Seite her in das Element 32 eingesetzt, wobei die Fahne 16 und ein Teil des Behälterkörpers 10 durch die Öffnung 37 des Elements 32 geführt werden. Dabei gelangen die Befestigungsnasen 33 des Elements 32 mit der Befestigungsnut 13 der Ampulle in Eingriff und halten diese in deren Längsrichtung. Im Anschluss wird der Deckel 30 wieder auf den Körper der Vorrichtung zu Aerosolerzeugung aufgesetzt. Durch Drehen des Deckels 30 und den Eingriff der Nasen des Deckels 30 mit den Gewindenuten des Elements 32 wird das Element 32 in der Öffnung 31 des Deckels 30 translatorisch bewegt. Da die Ampulle mit dem Element 32 verbunden ist, wird auch die Ampulle translatorisch bewegt. Dabei kommt zunächst, wie es in Fig. 5 dargestellt ist, eine Innenfläche des Kragens 15 mit dem O-Ring 38, der den Dorn 34 vollständig umgibt, in Eingriff und dichtet zwischen der Innenfläche des Kragens 15 und der Außenfläche des Dorns 34 zuverlässig ab. Wie es in Fig. 5 dargestellt ist, geschieht dies bevor die Schneidkante 35 des Dorns 34 mit dem Öffnungsvorgang, d. h. dem Durchtrennen der Sollbruchstelle 12, beginnt. Ferner wird die Ampulle durch die konische Innenkontur des Kragens 15 auf dem Dorn 34 zentriert. Durch diese Ausgestaltung wird zuverlässig verhindert, dass während des Öffnungsvorgangs Medikament entweichen kann. Darüber hinaus bleibt diese Abdichtung auch in voll eingesetztem Zustand (Fig. 6) bestehen, wobei die Abdichtung dann ggf. auch zwischen der Außenfläche des Dorns 34 und der Innenfläche des Behälterkörpers unmittelbar an oder oberhalb des Behälterbodens 11 stattfinden kann. Darüber hinaus wird ein zuverlässiges Einführen der Ampulle gewährleistet.

Durch eine weitere Drehbewegung des Deckels 30 und die damit verbundene weitere translatorische Bewegung des Elements 32 und somit der Ampulle wird der Behälterboden, wie es in Fig. 6 dargestellt ist, durch den Dorn 34 aufgestochen und zur Seite geklappt, so dass das in der Ampulle enthaltene Medikament durch den Dorn 34 zum Aerosolerzeuger 36 strömen kann. Dabei bewirkt die trichterförmige ausgestaltete Innenkontur des ersten Funktionsbereichs 20 des Behälterkörpers 10 ein Zuführen des Medikaments zum Aerosolerzeuger mit einer reproduzierbaren Dosisgenauigkeit. Darüber hinaus bewirkt die Anordnung der Befestigungsnut 13 in Längsrichtung der Ampulle mittig bzw. näher zum Behälterboden 12, dass die zum Öffnen der Ampulle erforderlichen Kräfte, die durch die Drehung des Deckels auf das Element 32 und damit die Ampulle übertragen werden möglichst nahe im Bereich der Krafteinleitung an der Sollbruchstelle 12 aufgenommen werden können. Im Übrigen versteifen die Verstärkungsrippen 14 die Ampulle derart, dass eine Deformierung beim Öffnungsvorgang im Wesentlichen vermieden wird.

Im vollständig eingesetzten Zustand sind die Öffnungen 37 des Elements 31, 32 des Deckels 30 im Wesentlichen in einer Ebene angeordnet. Auch in dieser Position ragt jedoch die Fahne 16 durch die Öffnung 37 des Elements 32 aus der Vorrichtung zu Aerosolerzeugung vor, so dass das Beschriftungsfeld 17 sowie die Chargennummer 18 und die nicht dargestellten Haltbarkeitsangaben auch im eingesetzten Zustand noch sichtbar sind.

Zum Entnehmen der Ampulle wird der Deckel 30 in der entgegengesetzten Richtung gedreht, wodurch das Element 32 in der entgegengesetzten Richtung wie beim Öffnen der Ampulle translatorisch bewegt wird und die Ampulle wieder vom Dorn 34 abzieht. Dabei bleibt die Abdichtung zwischen Ampulle (Kragen) und Dorn (O-Ring) so lange bestehen bis die Ampulle tatsächlich mit dem Deckel 30 vom Dorn 34 abgenommen werden kann, so dass auch hierbei eine Kontamination der Vorrichtung durch Medikamentenreste vermieden werden kann. Um die Ampulle aus dem Deckel 30 zu entfernen, kann der Anwender die Fahne 16 greifen und die Ampulle (in den Figuren nach unten) außer Eingriff mit den Nasen 33 drücken, so dass sie entnommen werden kann. Dabei muss der Anwender den ggf. durch ein Medikament benetzten Bereich der Ampulle nicht berühren, der zusätzlich vom Kragen 15 überdeckt wird. Des Weiteren kann bei besonders kurz ausgestaltetem zweitem Funktionsbereich 20 durch die zumindest vorragende Fahne 16 das Herausdrücken erleichtert werden.

Zusammenfassend bietet die vorliegende Erfindung damit eine Vielzahl von Vorteilen gegenüber der Ampulle des Standes der Technik. Es versteht sich jedoch, dass die dargestellte Ausführungsform nur eine Möglichkeit ist, die vorliegende Erfindung auszuführen und dass die Erfindung durch die folgenden Patentansprüche definiert ist.

Folgende jedoch nicht abschließen aufgelistete Wirkstoffklassen bzw. Substanzen können in der erfindungsgemäßen Ampulle enthalten sein:

Unter den wirksamen Verbindungen sind beispielsweise Substanzen, die aus der Gruppe ausgewählt sind, die aus antientzündlichen Verbindungen, Glucokorticoiden, Medikamenten gegen Allergien, Antioxidantien, Vitaminen, Leukotrin-Antagonisten, Mitteln gegen Infektionen, Antibiotika, Antifungiziden, Antivirusmitteln, Mucolytica, Dekongestiva, Antiseptika, Cytostatika, Immunmodulatoren, Impfstoffen, Mitteln zur Wundheilung, Lokalanästhetika, Oligonukleotiden, Peptiden, Proteinen und Pflanzenextrakten besteht.

Beispiele für möglicherweise nützliche anti-entzündliche Verbindungen sind Glucokorticoide und nicht-steroide anti-entzündliche Mittel, wie beispielsweise Betamethason, Beclomethason, Budesonid, Ciclesonid, Dexamethason, Desoxymethason, Fluoconolonacetonid, Flucinonid, Flunisolid, Fluticason, Icomethason, Rofleponid, Triamcinolonacetonid, Fluorcortinbutyl, Hydrocortison, Hydroxycortison-17-Butyrat, Prednicarbat, 6-Methylprednisolonaceponat, Mometasonfuroat, Dehydroepiandrosteronsulfat (DHEAS), Elastan, Prostaglandin, Leukotrin, Bradykinin-Antagonisten, nicht-steroide anti-entzündliche Medikamente (NSAIDs), wie beispielsweise Ibuprofen, einschließlich jeglicher pharmazeutisch annehmbarer Salze, Ester, Isomere, Stereoisomere, Diastereomere, Epimere, Solvate oder andere Hydrate davon, Promedikamente, Derivate oder irgendwelche anderen chemischen oder physikalischen Formen wirksamer Verbindungen, die die entsprechenden wirksamen Reste umfassen.

Beispiele für Mittel gegen Infektionen, deren Klasse oder therapeutische Kategorie hier als solche Verbindungen umfassend verstanden wird, die gegen bakterielle, pilzliche und virale Infektionen wirksam sind, d. h. solche, die die Klassen der Mikrobizide, Antibiotika, Fungizide, Antiseptika und Anti-Virus-Mittel umfassen, sind
- Penizilline, einschließlich Benzylpenizilline (Penizillin-G-Natrium, Clemizon-Penizillin, Benzathin-Penizillin G), Phenoxypenizilline (Penizillin V, Propizillin), Aminobenzylpenizilline (Ampizillin, Amoxyzillin, Bacampizillin), Acylaminopenizilline (Azlozillin, Mezlozillin, Piperazillin, Apalzillin), Carboxypenizilline (Carbenizillin, Ticarzillin, Temozillin), Isoxazolyl-Penizilline (Oxazillin, Cloxazillin, Dicloxazillin, Flucloxazillin) und Amiidin-Penizilline (Mecillinam);
- Cephalosporine, einschließlich Cefazoline (Cefazolin, Cefazedon); Cefuroxime (Cerufoxim, Cefamdol, Cefotiam), Cefoxitine (Cefoxitin, Cefotetan, Latamoxef, Flomoxef), Cefotaxime (Cefotaxim, Ceftriaxon, Ceftizoxim, Cefmenoxim), Ceftazidime (Ceftazidim, Cefpirom, Cefepim), Cefalexine (Cefalexin, Cefaclor, Cefadroxil, Cefradin, Loracarbef, Cefprozil) und Cefixime (Cefixim, Cefpodoxim-Proxetil, Cefuroxime-Axetil, Cefetamet-Pivoxil, Cefotiam-Hexetil), Loracarbef, Cefepim, Clavulansäure/Amoxizillin, Ceftobiprol;
- Synergisten, einschließlich Beta-Lactamase-Inhibitcren, wie beispielsweise Clavulansäure, Sulbactam und Tazobactam;
- Carbapeneme, einschließlich Imipenem, Cilastin, Meropenem, Doripenem, Tebipenem, Ertapenem, Ritipenam und Biapenem;
- Monobactame, einschließlich Aztreonam;
- Aminoglycoside, wie beispielsweise Apramycin, Gentamycin, Amikacin, Isepamicin, Arbekacin, Tobramycin, Netilmicin, Spectinomycin, Streptomycin, Capreomycin, Neomycin, Paromoycin und Kanamycin;
- Macrolide, einschließlich Erythromycin, Clarythromycin, Roxithromycin, Azithromycin, Dithromycin, Josamycin, Spiramycin und Telithromycin;
- Gyrase-Inhibitoren oder Flurochinolone, einschließlich Ciprofloxacin, Gatifloxacin, Norfloacin, Ofloxycin, Levofloxacin, Perfloxacin, Lomefloxacin, Garenoxacin, Clinafloxacin, Sitafloxacin, Prulifloxacin, Olamufloxacin, Caderofloxacin, Gemifloxacin, Balofloxacin, Trovafloxacin und Moxifloxacin;
- Tetracycline, einschließlich Tetracyclin, Oxytetracyclin, Rolitetracyclin, Minocyclin, Doxycyclin, Tigecyclin und Aminocyclin;
- Glycopeptide, einschließlich Vacomycin, Teicoplanin, Ristocetin, Avoparcin, Oritavancin, Ramoplanin und Peptide 4;
- Polypeptide, einschließlich Plectasin, Dalbavancin, Daptomycin, Oritavancin, Ramoplanin, Dalbavancin, Telavancin, Bacitracin, Tyrothricin, Neomycin, Kanamycin, Mupirocin, Paromomycin, Polymyxin B und Colistin;
- Sulfonamide, einschließlich Sulfadiazin, Sulfamethoxazol, Sulfalen, Co-Trimoxazol, Co-Trimetrol, Co-Trimoxazin und Co-Tetraxazin;
- Azole, einschließlich Clotrimazol, Oxiconazol, Miconazol, Ketoconazol, Itraconazol, Fluconazol, Metronidazol, Tinidazol, Bifonazol, Ravuconazol, Posaconazol, Voriconazol und Ornidazol und andere Antifungizide, einschließlich Flucytosin, Griseofluvin, Tonoftal, Naftifin, Terbinafin, Amorolfin, Ciclopiroxolamin, Echinocandin, wie Micafungin, Caspofungin, Anidulafungin;
- Nitrofurane, einschließlich Nitrofurantoin und Nitrofuranzon;
- Polyene, einschließlich Amphotericin B, Natamycin, Nystatin, Flucocytosin;
- andere Antibiotika, einschließlich Tithromycin, Lincomycin, clindamycin, Oxazolindione (Linzezolide), Ranbezolid, Streptogramin A+B, Pristinamycin aA+B, Virginiamycin A+B, Dalfopristin/Chiunupristin (Synercid), Chloramphenicol, Ethambutol, Pyrazinamid, Terizidon, Dapson, Prothionamid, Fosfomycin, Fucidinsäure, Rifampicin, Isoniazid, Cycloserin, Terizidon, Ansamycin, Lysostaphin, Iclaprim, Mirocin B17, Clerocidin, Filgrastim und Pentamidin;
- Antivirusmittel, einschließlich Aziclovir, Ganciclovir, Birivudin, Valaciclovir, Zidovudin, Didanosin, Thiacytidin, Stavudin, Lamivudin, Zalcitabin, Ribavirin, Nevirapirin, Delaviridin, Trifulridin, Ritonavir, Saquinavir, Indinavir, Foscarnet, Amantadin, Podophyllotoxin, Vidarabin, Tromantadin und Proteinase-Inhibitoren;
- Antiseptica, einschließlich Acridin-Derivate, Iod-Povidon, Benzoate, Rivanol, Chlorhexidin, quarternäre Ammonium-Verbindungen, Cetrimide, Biphenylol, Clorofen und Octenidin;
- Pflanzenextrakte oder Inhaltsstoffe, wie beispielsweise Pflanzenextrakte aus Kamille, Hamamelis, Echinacea, Calendula, Thymian, Papain, Pelagonien, Kiefernbäumen, ätherische Öle, Myrtol, Pinen, Limonen, Cineol, Thymol, Menthol, Kampfer, Tannin, Alpha-Hederin, Bisabolol, Lycopodin, Vitapherol;
- Verbindungen zur Wundheilung einschließlich Dexpantenol, Allantoin, Vitamine, Hyaluronsäure, Alpha-Antitrypsin, anorganische und organische Zinksalze/-verbindungen, Bismuth- und Selen-Salze;
- Interferone (Alpha, Beta, Gamma), Tumor-Nekrose-Faktoren, Cytokine, Interleukine;
- Immunmodulatoren einschließlich Methotrexat, Azathioprin, Cyclosporin, Tacrolismus, Sirolismus, Rapamycin, Mofetil; Mofetil-mycophenolat.
- Cytostatika und Metastase-Inhibitoren;
- Alkyliermittel, wie Nimustin, Melphanalan, Carmustin, Lomustin, Cyclophosphosphamid, Ifosfamid, Trofosamid, Chlorambucil, Busulfan, Treosulfan, Prednimustin, Thiotepa;
- Antimetabolite, z. B. Cytarabin, Fluoruracil, Methotrexat, Mercaptopurin, Thioguanin;
- Alkaloide, wie Vinblastin, Vincristin, Vindesin;
- Antibiotika, wie beispielsweise Alcarubicin, Bleomycin, Dactinomycin, Daunorubicin, Doxorubicin, Epirubicin, Idarubicin, Mitomycin, Plicamycin;
- Komplexe von Elementen der Übergangsgruppen (z. B. Ti, Zr, V, Nb, Ta, Mo, W, Pt) wie beispielsweise Carboplatin, Cis-Platin und Metallocenverbindungen, wie beispielsweise Titanocendichlorid;
- Amsacrin, Dacarbazin, Estramustin, Etoposid, Beraprost, Hydroxycarbamid, Mitoxanthron, Procarbazin, Temiposid;
- Paclitaxel, Iressa, Zactima, Poly-ADP-Ribose-Polymerase (PRAP)-Enzyminhibitoren, Banoxantron, Gemcitabin, Pemetrexed, Bevacizumab, Ranibizumab.

Beispiele möglicherweise nützlicher Mucolide sind DNase, P2Y2-Agnisten (Denufosol), Medikamente, die das Eindringen von Chlor- und Natrium betreffen, wie beispielsweise N-(3,5-diamino-6-chlorpyrazin-2-carbony)-N'-{4-[4-(2,3-dihydroxypropoxy)-phenyl]butyl}guanidin-Methanesulfonat (PARION 552-02) Heparinoide, Guaifenesin, Acetylcystein, Carbocystein, Ambroxol, Bromhexin, Tyloxapol, Lecithine, Myrtol und rekombinante Tensid-Proteine.

Beispiele potenziell nützlicher Vasokonstriktoren und Dekongestiva, die nützlich sein können, um die Schwellung der Schleimhaut zu reduzieren, sind Phenylephrin, Naphazolin, Tramazolin, Tetryzolin, Oxymetazolin, Fenoxazolin, Xylometazolin, Epinephrin, Isoprenalin, Hexoprenalin und Ephedrin.

Beispiele für potenziell nützliche Lokalanästhetika schließen Bezocain, Tetracain, Procain, Lidocain und Bupivacain ein.

Beispiele möglicher nützlicher Mittel gegen Allergien schließen die oben erwähnten Glucocorticoide, Cromolyn-Natrium, Nedocromil, Cetrizin, Loratidin, Montelukast, Roflumilast, Ziluton, Omalizumab, Heparinoide und andere Anthistaminika, einschließlich Azelastin, Cetirizin, Desloratadin, Ebastin, Fexofenadin, Levocetirizin, Loratadin ein.

Antisense-Oligonucleotide sind kurze synthetische Stränge aus DNA (oder Analoga), die komplementär oder im Gegensinn zur Zielsequenz (DNA, RNA) sind, welche so entworfen sind, dass sie einen biologischen Vorgang stoppen, wie beispielsweise die Transkription, Translation oder das Splicing. Die dadurch hervorgerufene Inhibierung der Genexpression macht Oligonucleotide, abhängig von ihrer Zusammensetzung, nützlich für die Behandlung vieler Erkrankungen, und zahlreiche Verbindungen werden derzeit klinisch untersucht, wie beispielsweise ALN-RSV01, um das respiratorische Syncytiumvirus zu behandeln, AVE-7279, um Asthma und Allergien zu behandeln, TPI-ASM8, um allergisches Asthma zu behandeln, 1018-ISS, um Krebs zu behandeln.

Beispiele potenziell nützlicher Peptide und Proteine schließen Aminosäuren ein, wie beispielsweise L-Arginin, L-Lysin, Antikörper gegen Toxine, die von Mikroorganismen hergestellt werden, anitmikrobielle Peptide, wie beispielsweise Cecropine, Defensine, Thionine und Cathelicidine.

Für jedes dieser und anderer explizit erwähnte Beispiele für Medikamentensubstanzen, die potenziell zur Durchführung der Erfindung nützlich sind, sollen die Verbindungsnamen, die hier angegeben worden sind, so verstanden werden, dass sie auch jegliche pharmazeutisch annehmbaren Salze, Solvate oder andere Hydrate, Promedikamente, Isomere oder irgendwelchen anderen chemischen oder physikalischen Formen der entsprechenden Verbindungen, die die entsprechenden aktiven Reste umfassen, betreffen.

## Patentansprüche

1. Einwegampulle zum Einsatz in eine Vorrichtung zur Aerosolerzeugung, umfassend:
einen Medikamentenbehälter, der ein Medikament enthält und aus einem Behälterkörper (10) und einem Behälterboden (11) gebildet ist,
eine den Behälterboden zumindest teilweise umgebende Sollbruchstelle (12), und
einen Kragen (15), der die Sollbruchstelle nach außen hin umgibt, wobei der Kragen den Behälterkörper derart über den Behälterboden hinaus verlängert, dass der Behälterboden und die Sollbruchstelle in einem Abstand von einer Stirnseite (19) der Einwegampulle sitzen und durch den Kragen geschützt sind.
**dadurch gekennzeichnet, dass** sich der Kragen (15) zumindest an seiner Innenkontur von dem Behälterboden (11) ausgehend konisch aufweitet.

2. Ampulle nach einem der vorstehenden Ansprüche, bei der der Behälterkörper in zwei Funktionsbereiche (20, 21) unterteilt ist, so dass die Ampulle modular aufgebaut ist.

3. Ampulle nach Anspruch 2, bei der die Ampulle im ersten Funktionsbereich (21), der den Behälterboden umfasst, zumindest an ihrer Innenkontur zum Behälterboden (11) hin konisch zulaufend ausgestaltet ist und der zweite Funktionsbereich (20) im Wesentlichen hohlzylindrisch ausgebildet ist.

4. Ampulle nach einem der vorstehenden Ansprüche, bei der eine Befestigungsnut (13) in Längsrichtung der Ampulle mittig oder näher zum Behälterboden (11) vorgesehen ist und bevorzugterweise die Ampulle in die zwei Funktionsbereiche (20, 21) unterteilt.

5. Ampulle nach einem der vorstehenden Ansprüche, bei der wenigstens eine Verstärkungsrippe (14) auf dem Behälterkörper (10) vorgesehen ist.

6. Ampulle nach einem der vorstehenden Ansprüche, bei der gegenüber dem Behälterboden (11) eine Fahne (16) an den Behälterkörper (10) vorgesehen ist.

7. Ampulle nach einem der vorstehenden Ansprüche, bei der die Ampulle einstückig ausgebildet ist.

8. Ampulle nach Anspruch 7, die im Blow-Fill-Seal Verfahren hergestellt ist.

9. Ampulle nach einem der vorstehenden Ansprüche, die aus Polyethylen, Polypropylen oder einem thermoplastischen Copolymer besteht.

10. Ampulle nach einem der vorstehenden Ansprüche, bei der in den Kragen (15) ein zusätzliches separates Dichtelement eingesetzt oder einsetzbar ist.

11. Ampulle nach einem der vorstehenden Ansprüche, bei der durch Dimensionierung und Ausgestaltung des Behälterbodens (11) sowie der Sollbruchstelle (12) ein Öffnungsdurchmesser von wenigstens 8 mm, vorzugsweise in einem Bereich zwischen 8 und 15 mm, am meisten bevorzugt von 10 mm erzielbar ist.

12. Ampulle nach einem der vorstehenden Ansprüche, bei der der Medikamentenbehälter bis zu 10 ml, vorzugsweise zwischen 0,25-5 ml Medikament enthält.

13. Ampulle nach einem der vorstehenden Ansprüche, bei der das Medikament wenigstens einen Wirkstoff enthält und vorzugsweise wenigstens einen Hilfsstoff in gelöster oder suspendierter Form.

14. Verwendung einer Ampulle nach einem der vorstehenden Ansprüche in einer Vorrichtung zur Aerosolerzeugung zur lokalen, nasalen oder pulmonalen Applikation, wobei die Vorrichtung eine perforierte schwingende Membran aufweist und das Medikament von der Membran zu Tröpfchen mit einem mittleren Massendurchmesser von kleiner 6 µm vernebelbar ist oder wobei das Medikament von der Vorrichtung als pulsierendes Aerosol mit einem mittleren Massendurchmesser von kleiner 6 µm in die Nase und Nasennebenhöhlen vernebelbar ist.

## Claims

1. Disposable ampoule for use in a device for aerosol generation comprising:
a medicament container which contains a medicament and is formed from a container body (10) and a container base (11),
a predetermined break point (12) at least partly surrounding the container base, and
a collar (15) which surrounds the predetermined break point outwardly, wherein the collar extends the container body beyond the container base such that the container base and the predetermined break point sit at a distance from an end-face side (19) of the disposable ampoule and are protected by the collar, **characterised in that** the collar (15) expands conically at least at its inner contour starting from the container base (11).

2. Ampoule according to one of the above claims, in which the container body is divided into two functional regions (20, 21) so that the ampoule is constructed in modular manner.

3. Ampoule according to claim 2, in which the ampoule is designed to be tapered towards the container base (11) in the first functional region (21), which comprises the container base, at least at its inner contour and the second functional region (20) is designed as an essentially hollow cylinder.

4. Ampoule according to one of the preceding claims, in which an attachment groove (13) is provided centrally or closer to the container base (11) in the longitudinal direction of the ampoule and the ampoule is preferably divided into the two functional regions (20, 21).

5. Ampoule according to one of the preceding claims, in which at least one reinforcing rib (14) is provided on the container body (10).

6. Ampoule according to one of the preceding claims, in which a lug (16) is provided on the container body (10) opposite the container base (11).

7. Ampoule according to one of the preceding claims, in which the ampoule is designed to be integral.

8. Ampoule according to claim 7, which is produced by the blow-fill-seal method.

9. Ampoule according to one of the preceding claims, which consists of polyethylene, polypropylene or a thermoplastic copolymer.

10. Ampoule according to one of the preceding claims, in which an additional separate sealing element is inserted or can be inserted into the collar (15).

11. Ampoule according to one of the preceding claims, in which an opening diameter of at least 8 mm, preferably in a range between 8 and 15 mm, most preferably of 10 mm, can be achieved by dimensioning and designing the container base (11) and the predetermined break point (12).

12. Ampoule according to one of the preceding claims, in which the medicament container contains up to 10 ml, preferably between 0.25-5 ml of medicament.

13. Ampoule according to one of the preceding claims, in which the medicament contains at least one active ingredient and preferably at least one adjuvant in dissolved or suspended form.

14. Use of an ampoule according to one of the preceding claims in a device for aerosol generation for local, nasal or pulmonary administration, wherein the device has a perforated oscillating membrane and the medicament can be nebulised by the membrane to form droplets having an average mass diameter of less than 6 µm or wherein the medicament can be nebulised by the device as a pulsating aerosol having an average mass diameter of less than 6 µm into the nose and paranasal sinuses.

## Revendications

1. Ampoule jetable destinée à être insérée dans un dispositif générateur d'aérosol, comprenant :
un conteneur de médicament qui contient un médicament et constitué d'un corps de conteneur (10) et d'un fond de conteneur (11), et
une ligne de cassure obligée (12) entourant au moins partiellement le fond de conteneur, et
une collerette (15) entourant par l'extérieur la ligne de cassure obligée, où ladite collerette prolongeant le corps de conteneur au-dessus du fond de conteneur de telle manière que le fond de conteneur et la ligne de cassure obligée sont espacés d'une face frontale (19) de l'ampoule jetable et sont protégés par la collerette,
**caractérisée en ce que**
la collerette (15) s'élargit coniquement depuis le fond de conteneur (11), au moins sur son contour intérieur.

2. Ampoule selon l'une des revendications précédentes, où le corps de conteneur est divisé en deux zones fonctionnelles (20, 21), de manière à donner une structure modulaire à l'ampoule.

3. Ampoule selon la revendication 2, où, dans la première zone fonctionnelle (21) qui comprend le fond de conteneur, ladite ampoule est réalisée de manière à converger coniquement vers le fond de conteneur (11), au moins sur son contour intérieur, et où la deuxième zone fonctionnelle (20) est réalisée sensiblement comme un cylindre creux.

4. Ampoule selon l'une des revendications précédentes, où une rainure de fixation (13) est prévue centralement dans la direction longitudinale de l'ampoule où plus à proximité du fond de conteneur (11), et où l'ampoule est préférentiellement divisée en deux zones fonctionnelles (20, 21).

5. Ampoule selon l'une des revendications précédentes, où au moins une ailette de renforcement (14) est prévue sur le corps de conteneur (10).

6. Ampoule selon l'une des revendications précédentes, où un onglet (16) est prévu sur le corps de conteneur (10), opposé au fond de conteneur (11).

7. Ampoule selon l'une des revendications précédentes, où ladite ampoule est réalisée en une seule pièce.

8. Ampoule selon la revendication 7, fabriquée avec le procédé Blow-Fill-Seal.

9. Ampoule selon l'une des revendications précédentes, en polyéthylène, polypropylène ou en copolymère thermoplastique.

10. Ampoule selon l'une des revendications précédentes, où un élément d'étanchéité complémentaire séparé est mis en place, ou peut être mis en place dans la collerette (15).

11. Ampoule selon l'une des revendications précédentes, où un diamètre d'ouverture d'au moins 8 mm, préférentiellement compris entre 8 et 15 mm, et plus particulièrement égal à 10 mm, peut être obtenu par le dimensionnement et la conformation du fond de conteneur (11) et de la ligne de cassure obligée (12).

12. Ampoule selon l'une des revendications précédentes, où le conteneur de médicament contient jusqu'à 10 ml, préférentiellement de 0,25 à 5 ml de médicament.

13. Ampoule selon l'une des revendications précédentes, où le médicament contient au moins une substance active et préférentiellement au moins un excipient sous forme de solution ou de suspension.

14. Utilisation d'une ampoule selon l'une des revendications précédentes dans un dispositif générateur d'aérosol pour une administration locale, nasale ou pulmonaire, dans lequel ledit dispositif présente une membrane vibrante perforée et le médicament est nébulisable par la membrane en gouttelettes ayant un diamètre massique moyen inférieur à 6 µm, ou dans lequel le médicament est nébulisable par le dispositif sous forme d'aérosol pulsé ayant un diamètre massique moyen inférieur à 6 µm dans le nez et les sinus paranasaux.
